# EUROPEAN PATENT SPECIFICATION

(11) **EP 1 309 293 B1**
(45) Date of publication and mention of the grant of the patent: **23.08.2006**
(21) Application number: 01975287.2
(22) Date of filing: 21.09.2001
(51) Int. Cl.: A61F 2/06

(54) **INTRAVASCULAR STENT APPARATUS**
INTRAVASCULÄRER STENT
STENT INTRAVASCULAIRE

(30) Priority: 22.09.2000 US 234614 P
(43) Date of publication of application: 14.05.2003
(60) Divisional application: 06010357.9
(73) Proprietor: Boston Scientific Scimed, Inc., Maple Grove, MN 55311 (US)
(72) Inventor: Jang, G. David, Redlands, CA 92374 (US)
(74) Representative: Hauck, Graalfs, Wehnert, Döring, Siemons
(86) International application number: PCT/US2001/029562
(87) International publication number: WO 2002/024112

(56) References cited:
- WO-A-00/62710
- WO-A-02/24112
- WO-A-97/40780
- WO-A-98/20810
- US-A- 5 733 303

## Description

### BACKGROUND OF THE INVENTION

### Field of Invention:

This invention relates to intravascular stents in general, and more particularly to intracoronary stents.

### Description of the Related Art:

Intracoronary stents provide intraluminal scaffolding support of the vascular wall after percutaneous angioplasty in which the balloon catheter is used to expand the stenotic vascular lesion. In both the delivery phase and the deployed phase, there are numerous performance factors that can characterize the overall clinical performance of a stent and can be improved.

By the year 2000, the percutaneous balloon angioplasty and stent implant procedures have become the dominant non-surgical revascularization method of the atherosclerotic stenosis, or obstruction, of the vascular lumen, and particularly in the coronary vascular system of the heart. With balloon angioplasty alone and without stents, the restenosis rate after angioplasty has been as high as 25-45% in the first time coronary cases. With stents after balloon angioplasty, the restenosis rate has been reduced significantly. Even so, the restenosis rate after stent implantation is reported to be 15-25% range in coronary arteries, depending on the condition of the stented vessel or the specific stent. An ideal coronary stent is still elusive in the current state of the art commercial products.

Some of the best selling current, second generation, stents can be divided into two categories. One category is a stent with high flexibility and the other category has full vessel coverage. The flexible stents generally have poor vessel coverage, tissue prolapse, rough surface modulation and increased restenosis rate. On the other hand, a stent with good vessel coverage in the current state of art may not be flexible enough for easy delivery and for highly efficient procedures. This means that a stent with good flexibility and good vessel coverage remains as the unfulfilled gold standard.

To further reduce the restenosis rate after stent implant, numerous means have been tried including laser, atherectomy, high frequency ultrasound, radiation device, local drug delivery, etc. Although the brachytherapy (radiation treatment) has proved to be reasonably effective in further reducing restenosis after stent implant, using brachytherpy is very cumbersome, inconvenient, and costly. Brachytherapy is a radioactive device and a radiation therapy specialist from another department has to be involved with the interventional cardiologist in the cardiac catheterization laboratory. The laser and atherectomy devices proved to be marginally useful with added costs.

Local drug therapy appears to be a very promising method for the future, as better pharmaceutical, chemical, or biogenetic agents are developed and became available. Some research data, both from animal tests and human clinical studies indicate evidence of some suppression of restenosis after stent implantation when certain growth blocking pharmaceutical agents coat the stent. In other instances, it has been speculated that certain surface modifying materials coated on the surface of the stent may be beneficial, alone or in combination with growth suppressing agents, in reducing the restenosis rate. In either instance, a drug or substance should be locally attached or coated on the stent in sufficient amounts. However, attaching or coating a sufficient amount of a substance or drug on the coronary stent may not be an easy proposition, because coating enough volume of the drug on the small surface area of a stent is a challenging task. If and when stent coating becomes practical, a good stent can still have better outcomes than a poorly designed stent when used with substance coating.

A stent is a scaffolding device. When delivered to a remote vessel location via percutaneous approach it can be deployed by expanding the device inside a vessel. The vessel can have a very small caliber and sometimes has a very tortuous anatomy. When a stent is deployed, the stent should have a good radial strength, a good vessel coverage, a good internal surface modulation without tulips (i.e., sharp metal loop projections that resemble fish scale phenomena), an optimal vessel conformability, a low metal fraction, and so forth. If the stent is stiff and non-flexible, it can be very difficult to deliver to an intended lesion site inside a vessel. Easy delivery of a stent is aided by good flexibility of the stent in combination with the delivery balloon, a smooth surface modulation without or minimizing tulips and a degree of radiopacity. A good stent should have a combination of features for delivery and deployment.

Although there are countless variations of vascular stent designs today, few have these desired stent features both in the delivery phase and in the post-delivery phase. Today's top selling stents in the market can have undesirable characteristics, either in the delivery phase or in the deployed phase of the stent life cycle. For example, some stents may have flexibility, but lack vessel coverage or surface modulations both in delivery and deployed phases. Some stents may have good vessel coverage and surface modulations, but lack flexibility.

Vascular stents, which are designed to be delivered to vessel sites via percutaneous approach, can have two elements. The first element is the expansion strut that expands circumferentially to provide the scaffolding radial force against a possible collapsing force of the vessel wall. The second element is the connecting strut that can link the expansion struts along the longitudinal axis of the stent, giving articulation or flexibility to the stent. The particular combination of expansion struts and connecting struts generally form various cells, depending on the specific configuration and shape of the expansion and connecting struts. If a cell is too large, the vessel wall support or coverage can be poor and the vessel wall tissue can prolapse through the large cells of the stent net. If the cells are too small, the vessel wall may be well covered but the metal fraction of the stent can be too high. The metal fraction is a fraction of the total metal surface area of an expanded stent (inside a blood vessel) divided by the total internal vessel wall surface area where the stent is deployed.

Some very flexible stents have very large cell size with poor vessel coverage and tissue prolapse, in addition to poor (inner and/or outer) surface modulation due to large numbers of tulips directed to both ends of the stent. Most of the current flexible stents are designed to effect flexibility by using fewer or a minimal number of connecting struts, handicapping the vessel coverage, surface modulation and tissue prolapse defects.
On the other hand, a stent that is designed for good vessel coverage and ideal cell size tends to be inflexible when such a stent is being delivered to a vessel lesion. A lack of flexibility during stent delivery is a very critical issue ; a stiff stent often cannot be delivered to a needed location inside a blood vessel because such a stent cannot navigate through a tortuous and small vessel lumen. There is a need for a vascular stent that is very flexible for delivery and with good vessel coverage when deployed.

US 5,733,303 describes a flexible expandable stent having expansion strut columns connected by individual connecting struts. The connecting struts connect the proximal end of an expansion pair and a first expansion column to the distal end of a expansion pair in a second expansion column. Each connecting strut has five sections three of which in a longitudinal direction to a radial direction.

WO 98/20810 shows a flexible, radially expansible luminal protheses having expansion columns connected to each other by connecting struts. The expansion struts of the expansion columns form expansion strut pairs having a v-like shape. Adjacent expansion strut pairs have one expansion strut in common and point in opposite directions. A straight connecting strut connects a distal end of a connecting strut pair and a first column to a proximal end of a connecting strut pair in the second column or vise versa.

The technical problem of the invention is to provide a vascular stent being very flexible for delivery and with good vessel coverage when deployed in particular showing a uniform distribution of the force.

The problem is solved by stent according to claim 1.

### BRIEF DESCRIPTION OF DRAWINGS

Figure 1 shows a side elevation view of an embodiment of a stent, such as a tubular stent.
Figure 2 shows an isometric view of an embodiment of a stent, such as a tubular stent.
Figure 3 shows a cut-open view of an embodiment of a stent. Various expansion columns and connecting strut columns are shown.
Figure 4 shows a magnified view of a middle section of an embodiment of a stent, such as a stent of Figures 1, 2, and/or 3. Some details are shown of expansion columns.
Figure 5 shows a magnified view of a middle section of an embodiment of a stent, such as a stent of Figures 1, 2, and/or 3. Some details are shown of connecting strut columns.

### DETAILED DESCRIPTION OF DRAWINGS

Some embodiments of stents can be in a state, such as one or more of a non-expanded state, an expanded state, a crimped state, and a non-crimped state.

Some embodiments of stents can include one or more of a first expansion column, a second expansion column, a third expansion column, a first connecting strut column, and a second connecting strut column.

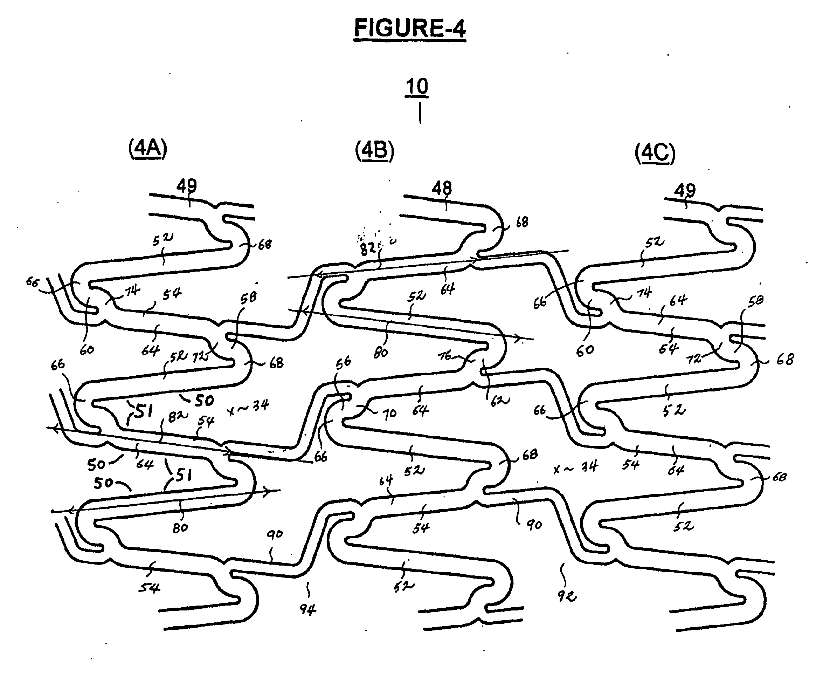

The first expansion column, the second expansion column, and/or the third expansion column can include individual expansion struts forming a plurality of expansion strut pairs. Figure 4 shows examples of individual expansion struts 50 forming a plurality of expansion strut pairs 51. In some embodiments of the stent, one expansion strut of an expansion strut pair has a stair-step segment at a proximal end *or* a stair-step segment at a distal end. The other expansion strut of the expansion strut pair can be a straight segment. Figure 4 shows examples of one expansion strut 54 of an expansion strut pair having a stair-step segment at a proximal end or a stair-step segment at a distal end. Figure 4 also shows examples of the other expansion strut 52 of the expansion strut pair being a straight segment. In some embodiments of the stent, distal ends of expansion strut pairs of the first expansion column are coupled to proximal ends of expansion strut pairs of the second expansion column in a vertically offset fashion. In many embodiments of the stent, two adjacent expansion strut pairs share a common strut.

The first connecting strut column and the second connecting strut column include a plurality of individual connecting struts. The first connecting strut column can include individual first connecting struts and the second connecting strut column can include individual second connecting struts. The individual first connecting column couples the first and second expansion columns. The second connecting column couples the second and third expansion columns.

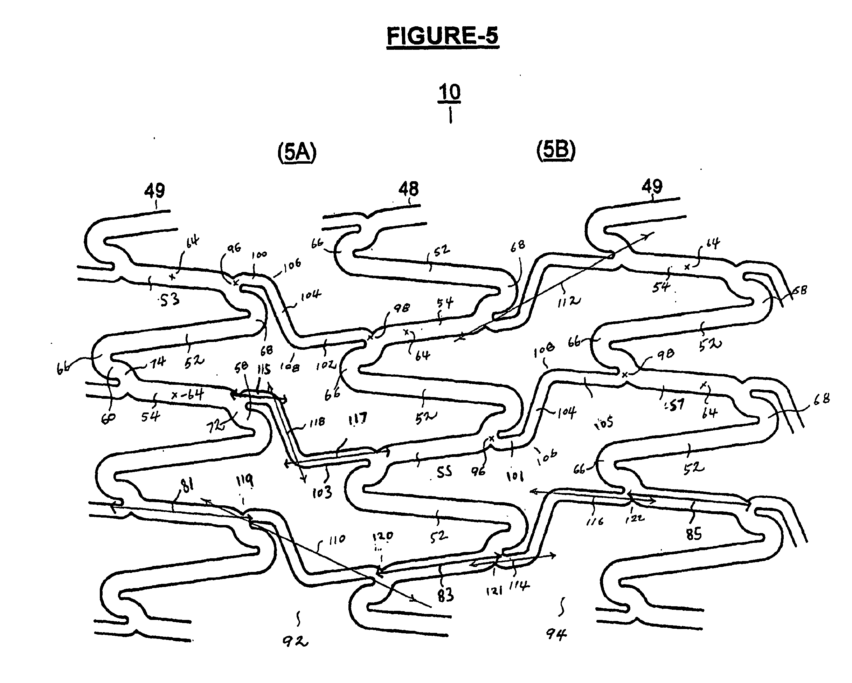

In various embodiments of the stent, each connecting strut can have a stair-step configuration, at least some number of pivot points, a same longitudinal axis as other connecting struts in the same connecting column, and various sections such as a proximal section, an intermediate section, and a distal section. A connecting strut can have at least two pivot points. Each pivot point can include a radius of curvature. Figure 5 shows examples of pivot points having radii of curvature 106 and 108. The longitudinal axes of connecting struts in a connecting column have the same direction. Figure 5 shows examples of a longitudinal axis 110 of a connecting strut which has the same longitudinal axis as other connecting struts in the same column, and a longitudinal axis 112 which has the same longitudinal axis as other connecting struts in the same connecting column.

In various embodiments of the stent, the proximal section of each first connecting strut in a connecting column has a terminal end 96 conjoined to an expansion strut in the first expansion column. A surface of the connecting strut can be conjoined to at least one surface of an expansion strut in the first expansion column. An edge of a connecting strut can be a linear extension of an edge of an expansion strut in the first expansion column. A strain relief notch can be formed where the edge of the proximal section is conjoined with the edge of the expansion strut of the first expansion column. Figure 5 shows an example of a strain relief notch 119 formed where the edge of the terminal end 96 of the proximal section is conjoined with the edge of the expansion strut of the first expansion column. At least one proximal section of a connecting strut can be a direct extension of an expansion strut pair of the first expansion column. Figure 5 shows an example of a proximal section 100 which can be a direct extension of an expansion strut pair that includes expansion strut 53. The longitudinal axis can be non-parallel to the longitudinal axis of the distal section. Figure 5 shows an example of a longitudinal axis 115 of a proximal section is non-parallel to the longitudinal axis 117 of the distal section. The longitudinal axis can be parallel to the longitudinal axis of an expansion strut in the first expansion column. Figure 5 shows an example of a longitudinal axis 115 of a proximal section, which is parallel to the longitudinal axis 82 of an expansion strut of the first expansion column. The width of a connecting strut column in a connecting strut can be less than a width of the expansion strut in an expansion column. Figure 5 shows an example of a width of a proximal section 100, which is less than a width of an expansion strut 54 of the first expansion column.

In various embodiments of the stent, the proximal section of each second connecting strut can include one or more of: a terminal end conjoined to an expansion strut in the second expansion column, a surface conjoined to at least one surface of an expansion strut in the second expansion column, an edge that is a linear extension of an edge of an expansion strut in the second expansion column, a longitudinal axis, and a width. A strain relief notch can be formed where the edge of the proximal section is conjoined with the edge of the expansion strut of the second expansion column. Figure 5 shows an example of a strain relief notch 121 formed where the edge of the proximal section is conjoined with the edge of the expansion strut of the second expansion column. At least one proximal section can be a direct extension of an expansion strut pair of the first expansion column and/or the second expansion column. Figure 5 shows an example of a proximal section 101, which is a direct extension of either expansion strut pair including expansion strut 55. The longitudinal axis can be non-parallel to the longitudinal axis of the distal section. Figure 5 shows an example of a longitudinal axis 114, which is non-parallel to the longitudinal axis 116 of the distal section. The longitudinal axis can be parallel to the longitudinal axis of an expansion strut in the second expansion column. Figure 5 shows an example of a longitudinal axis 114, which is parallel to the longitudimal axis 83 of an expansion strut of the second expansion column. The width can be less than a width of the expansion strut of the second expansion column. Figure 5 shows an example of a width of a proximal section 101, which is less than a width of an expansion strut 55 of the second expansion column.

In various embodiments of the stent, the distal section 102 of a connecting strut can include a terminal end 98 conjoined to an expansion strut in the second expansion column. A surface of a connecting strut can be conjoined to an end of an expansion strut in the second expansion column. An edge of a connecting strut can be a linear extension of an edge of an expansion strut in the second expansion column. A strain relief notch can be formed where the edge of the distal section 102 is conjoined with the edge of the expansion strut of the second expansion column. Figure 5 shows an example of a strain relief notch 120 formed where the edge of the distal section 102 is conjoined with the edge of the expansion strut of the second expansion column. At least one end of a connecting strut can be a direct extension of an expansion strut pair of the first expansion column or the second expansion column. Figure 5 shows an example of a distal section 102, which is a direct extension of an expansion strut pair that includes an expansion strut 54. Figure 5 shows an example of a longitudinal axis 117, which is parallel to the longitudinal axis 83 of an expansion strut of the second expansion column. The width of a connecting strut can be less than a width of the expansion strut of the second expansion column. Figure 5 shows an example of a width of a distal section 103, which is less than a width of an expansion strut 54 of the second expansion column. The length of the distal section of a connecting strut can be greater than a proximal section. Figure 5 shows an example of a distal section 102 having a length greater than a proximal section 100.

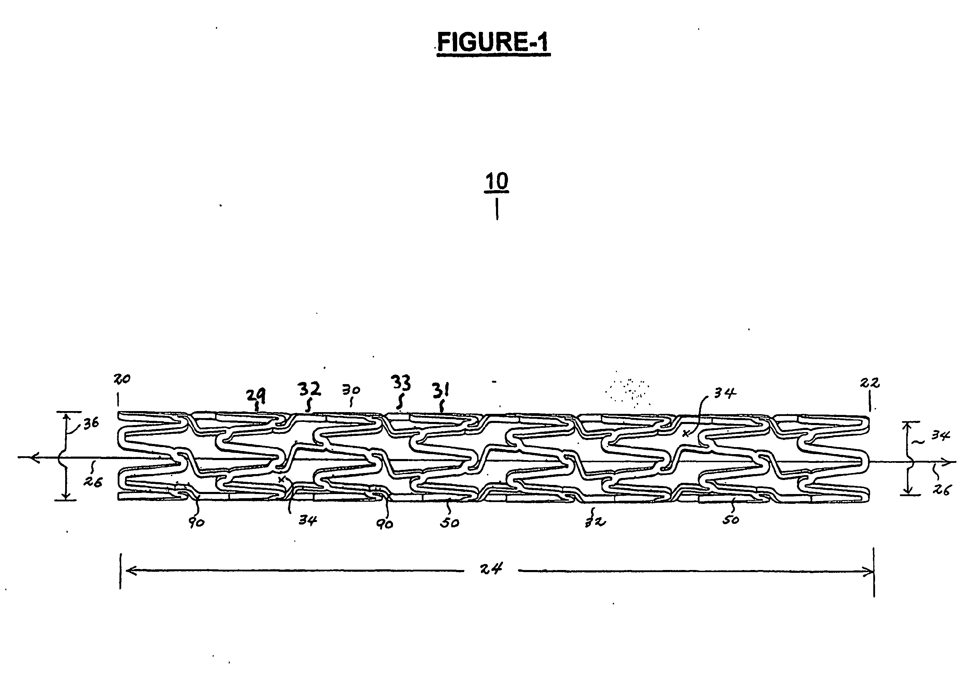

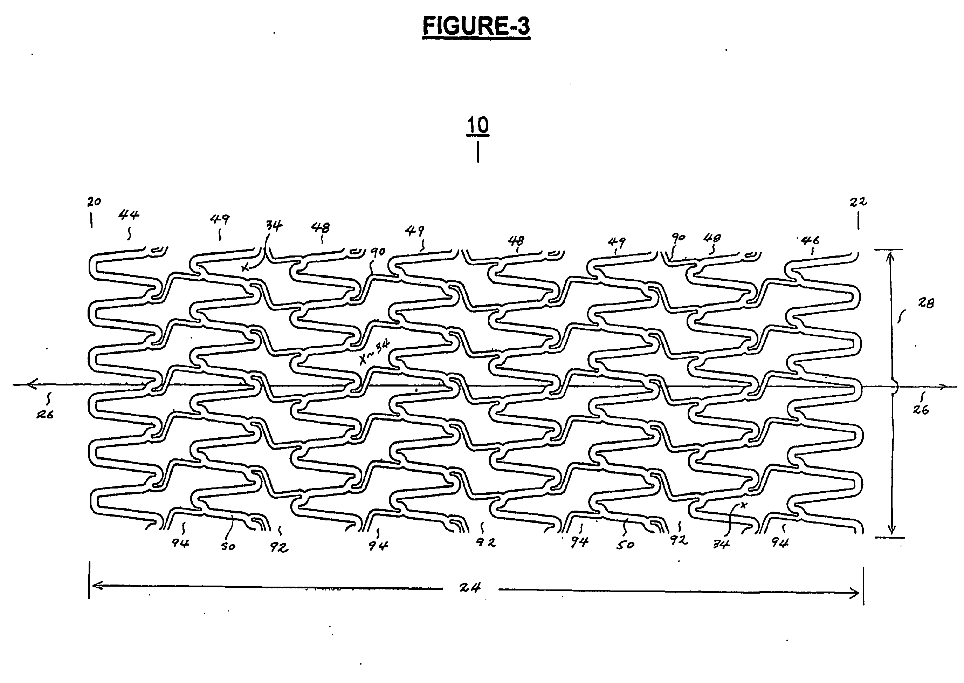

In various embodiments of the stent, the distal section of each second connecting strut can include one or more of: a terminal end conjoined to an expansion strut in the third expansion column, at least one surface conjoined to an end of an expansion strut in the third expansion column, an edge that is a linear extension of an edge of an expansion strut in the third expansion column, a longitudinal axis, a width, and a length. A strain relief notch can be formed where the edge of the distal section is conjoined with the edge of the expansion strut of the third expansion column. Figure 5 shows an example of a strain relief notch 122 formed where the edge of the distal section is conjoined with the edge of the expansion strut of the third expansion column. The distal section can be a direct extension of an expansion strut pair of the third expansion column. Figure 5 shows an example of a distal section 105, which is a direct extension of either expansion strut pair including expansion strut 57.
The longitudinal axis can be parallel to the longitudinal axis of an expansion strut in the third expansion column. Figure 5 shows an example of a longitudinal axis 116, which is parallel to the longitudinal axis 85 of an expansion strut of the third expansion column. The width can be less than a width of the expansion strut of the third expansion column. Figure 5 shows an example of a width of a distal section 105, which is less than a width of an expansion strut 57 of the third expansion column. The length can be greater than a proximal section. Figure 5 shows an example of a distal section 105 having a length geater than a proximal section101. In various embodiments of the stent, the intermediate section 104 of each second connecting strut can be coupled to the proximal and distal sections of a second connecting strut, and the intermediate section 104 have a length greater than a length of the proximal section 100 of a second connecting strut.
The longitudinal axis of the intermediate section extends in a vertically diagonal direction relative to a longitudinal axis of the stent and is non-parallel to a longitudinal axis of the stent. At least a portion of the intermediate section is placed in close proximity to an expansion strut pair of the second expansion column. For example, close proximity can be in the range of 0.003 cm to 0.127 cm (0.001 to 0.050 of an inch), in the range of 0.003 cm to 0.127 cm (0.001 to 0.040 of an inch), and/or in the range of (0.001 to 0.030 of an inch) 0.003 cm to 0.127 cm.
In various embodiments of the stent, the intermediate section 104 of each first connecting strut in a first connecting column is coupled to the proximal and distal sections of the first connecting strut, and has a length greater than a length of the proximal section of the first connecting strut. The longitudinal axis of the intermediate section extends in a vertically diagonal direction relative to a longitudinal axis of the stent and is non-parallel to a longitudinal axis of the stent.
The diagonal direction of the longitudinal axis of the intermediate section of a second connecting strut in a second connecting column extends in an opposing direction of the diagonal direction of the longitudinal axis of the intermediate section of the first connecting strut in a first connecting column. At least a portion of the intermediate section is placed in close proximity to an expansion strut pair of the first expansion column. For example, close proximity can be in the range of 0.003 cm to 0.127 cm (0.001 to 0.050 of an inch), in the range of 0.003 cm to 0.106 cm (0.001 to 0.040 of an inch), and/or in the) range of 0.003 to 0.076 cm (0.001 to 0.030 of an inch.
In various embodiments of the stent, first connecting struts and second connecting struts can be conjoined to the first and second expansion columns on contralateral sides. In various embodiments of the stent, at least a portion of proximal and distal sections of first connecting struts and second connecting struts can have a curvilinear geometric configuration, particularly near a radius of curvature. In various embodiments of the stent, at least a portion of first connecting struts and second connecting struts can have asymmetrical or symmetrical geometric configurations. In various embodiments of the stent, all first connecting struts and all second connecting struts have parallel longitudinal axes.
In some embodiments of the stent, both terminal ends of the first connecting strut are conjoined to an expansion strut in the first expansion column and the second expansion column. Some embodiments of the stent include a plurality of expansion columns coupled by a plurality of connecting strut columns.
Some embodiments of the stent include a first end expansion column and a second end expansion column. The first end expansion column and the second end expansion column can define a proximal and a distal end of the stent, and they are mirror images to each other. For example, Figure 3 shows end expansion columns 44 and 46 as mirror images.
Some embodiments of the stent include a plurality of cells. The plurality of cells is defined by the first expansion column, the second expansion column and the first connecting strut column. The cells in the stent have asymmetrical geometry, but the cells also can have symmetrical geometry. The cells have evenly spaced geometry and the cells transform into a quasihexagonal geometry in a nominally expanded state. For example, Figures 1-5 shows cells 34 in a non-expanded form.

An expansion column of the stent has expansion struts in a ring configuration made of zigzag shaped expansion strut pair cycles. Expansion columns are responsible for radial expansion, optimal crimping, and radial strength of the stent. The expansion columns by themselves do not provide flexibility. Each zigzag cycle in an expansion column can have plurality of expansion strut pairs conjoined by a joining strut loop at either a proximal end or a distal end. This sequence continues, for twelve times in one embodiment, seamlessly around the circumference of an expansion column in a stent.

Various embodiments of the stent include one or more of several different types of expansion columns including one or more of several types of expansion struts. An end expansion column at the proximal end can include straight-line expansion struts and stair-step expansion struts with a short stepped-down segment at a distal end. An end expansion column at the distal end can include straight-line expansion struts and stair-step expansion struts with a short stepped-down segment at a proximal end. The end expansion columns can be mirror images. Terminating side of end expansion columns can have smooth and evenly rounded loops.

The middle of the stent can include, for example, alternating different types of expansion columns that can be mirror images. One expansion column type includes straight-line expansion struts and stair-step expansion struts with a stepped-down segment in the proximal end and a stepped-up segment in the distal end. Another expansion column type includes straight-line expansion struts and stair-step expansion struts with a stepped-up segment in the proximal end and a stepped-down segment in the distal end.

A stepped-down or stepped-up segment can be short in length near a proximal or a distal end of a long straight segment of a stair-step expansion strut and a short sloped transitional segment. A transitional segment of an expansion strut conjoins a connecting strut with an expansion strut, for example, with the long straight segment of the stair-step expansion strut. The connecting strut can be a direct extension of the expansion strut and be integral to the stent structure rather than a separate structure added, welded or attached. Separate terminology for stent elements, for example expansion and connecting struts, conveniently describes the anatomy and function of various stent portions.

A connecting strut in a connecting strut column is not directly conjoined to another connecting strut in the same connecting strut column or in another adjacent connecting strut column.

Proximal and distal ends of a stair-step connecting strut can be a direct extension of an expansion strut pair loop of adjacent expansion columns. A stair-step connecting strut in a connecting strut column can inter-connect two apposed expansion strut pair loops in a vertically offset, diagonal direction. Expansion strut pair loops of two adjacent expansion columns can be arranged in a peak-to-valley apposition.

A stair-step connecting strut in a connecting strut column can have a short segment on an end directly extending from an expansion strut pair of an expansion column and a longer segment on the other end directly extending from an expansion strut pair of an adjacent expansion column. Between these two end segments a connecting strut can include a straight center segment having a slant-angle orientation relative to the two end segments. A center segment can be placed in close proximity of an expansion strut pair loop of one of two adjacent expansion columns, which are conjoined by the connecting strut.

A stair-step connecting strut can have each end conjoined on the ipsilateral sides of apposed expansion strut pair loops of adjacent expansion columns. A longitudinal axis of a stair-step connecting strut can have a diagonal orientation relative to the longitudinal axis of the stent A diagonally oriented axis of a stair-step connecting strut in one connecting strut column has a first direction, and a diagonally oriented axis of a stair-step connecting strut in an adjacent connecting strut column has a second direction. Longitudinal axes of connecting struts in adjacent connecting strut columns can run in opposing directions.

A connecting strut can have three straight segments, two pivot points with two radii of curvature, and two ends that extend directly from expansion struts of adjacent expansion columns. The pivot points in a connecting strut can serve as flexing points for stent flexibility. Longitudinal axes of horizontally oriented segments of connecting strut can run in a same direction as a longitudinal axis of a stair-step expansion strut. A central segment of a connecting strut may not be parallel with horizontally oriented segments of the connecting strut. A longitudinal axis of a centrally located intermediate segment of a connecting strut can have a diagonal orientation to longitudinal axes of horizontally oriented segments of the connecting strut and to the longitudinal axis of the stent.

A connecting strut column can conjoin adjacent expansion columns forming enclosed stent cells of asymmetrical geometry. Cells can transform into a roughly hexagonal geometry when, for example, the stent is nominally expanded in a 3-dimensional tubular state.

Some embodiments of the stent include a first expansion column, a second expansion column, and a first connecting strut. The first expansion column and the second expansion column can include expansion struts forming a plurality of expansion strut pair loops. Expansion strut pair loops can couple adjacent expansion struts. Two adjacent expansion struts can share a common strut The first connecting strut column can include a plurality of individual connecting struts.

Figure 1 shows one embodiment of a stent 10 in side elevation view, with a first expansion column 29, a second expansion column 30, a third expansion column 31, a first connecting strut column 32, and a second connecting strut column 33. The stent 10 has a proximal end 20 and a distal end 22. The stent 10 can have a tubular or cylindrical structure. The stent 10 can have a longitudinal length 24 and a longitudinal axis 26.

In some embodiments of the stent, an expansion column can be a zigzag and/or corrugated ring configuration of expansion struts. An expansion column, for example expansion column 30, in a stent 10 can be an unbroken circular ring. Multiple expansion strut columns can be interconnected with connecting struts continuously along the longitudinal axis 26 of the stent 10 in an unbroken manner to form a stent 10 having a tubular shape. The interconnections among expansion columns and connecting strut columns enclose spaces, or cells, formed by expansion struts and connecting struts. In the embodiment shown in Figure 1, many cells have asymmetrical geometry. The stent 10 has two different diameters, including an outer diameter 36 and an inner diameter 38, having a difference of a thickness of the stent 10. Both the outer diameter 36 and inner diameter 38 can change as the stent 10 goes through a crimping stage, when the diameters 36 and 38 are narrowed, and through a deployed stage, when the diameters 36 and 38 are expanded.

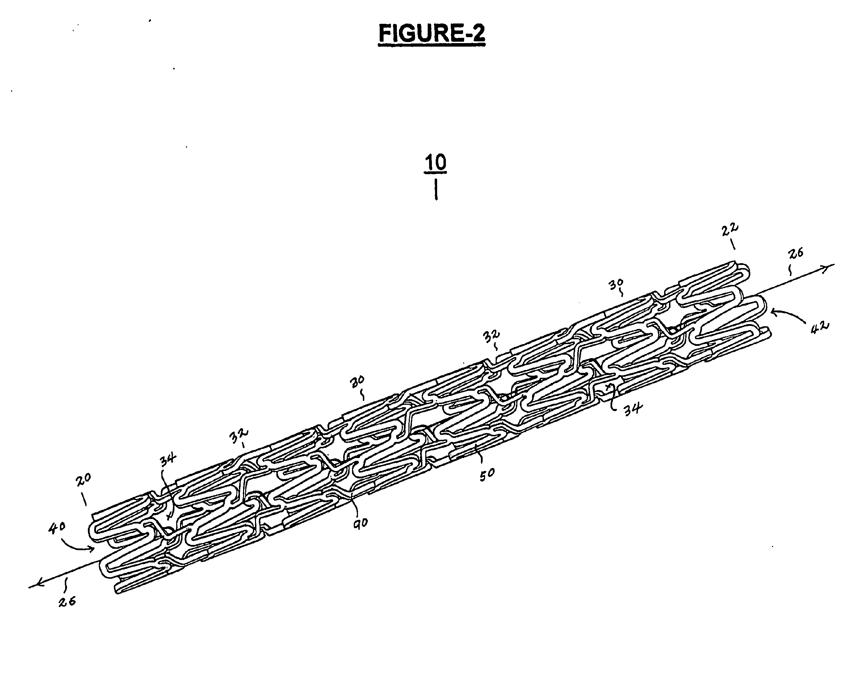

Figure 2 shows one embodiment of a stent 10 in isometric view. A back half of the stent 10 can be seen through the front half of the stent 10. The shown embodiment of the stent 10 has a tubular structure with a central lumen, a proximal opening 40, and a distal opening 42. Stent cells 34 include open spaces in the network of expansion struts and connecting struts. The lumen includes the central, open tunnel formed by the stent.

Figure 3 shows one embodiment of a stent 10 in cut-open view. The stent 10 has a proximal end 20 and a distal end 22. This view of the stent 10 is a scale drawing for a 15 mm coronary stent. There are eight expansion columns and seven connecting strut columns. At the proximal end 20 is an expansion column 44, which is a mirror image of an expansion columns 46 at the distal end 22. In the middle of the stent 10, there are six expansion columns, such that an expansion column 49 alternates with an expansion column 48. Interconnecting with eight expansion columns along the longitudinal axis 26 of the stent 10 are seven connecting strut columns including four connecting strut columns 94 and three connecting strut columns 92, such that a connecting strut column 94 alternates with a connecting strut column 92. In the middle of the stent 10 are two upright connecting strut columns 132 and three upside down connecting strut columns 134. There are a total of 42 cells of four different asymmetric configurations. All the cells have asymmetrical geometry. Expansion columns 44, 46, 48, and 49 are vertically arranged with expansion strut pair loops aligned peak-to-valley. Connecting strut columns 92 and 94 interconnect expansion columns 44, 46, 48, and 49 in a continuous and unbroken manner along the length 24 and around the circumference 28 of the stent 10.

The stent 10 in Figure 3 has the proximal end 20 on the left and the distal end 22 on the right. The stent 10 has a length 24 horizontally and a circumference 28 vertically, with a longitudinal axis 26 horizontally along the length 24 from the proximal end 20 to the distal end 22.

A width (horizontal dimension) of expansion columns is wider than a width of connecting strut columns. However, a width of a connecting strut column could be made the same or larger than a width of an expansion column. The variation of width ratio between a connecting strut column and an expansion column are within the scope of present invention of stent 10. The number of expansion strut cycles in an expansion column and the number of connecting struts in a connecting strut column can be made variably different. Variable numbers of making expansion strut cycles and connecting struts are within the scope of the present invention of the stent 10.

Figure 4 shows a magnified view of a middle section of one embodiment of a stent 10. Figure-4 shows several expansion columns 48 and 49. Each expansion column can have six cycles of continuous, unbroken expansion strut pair loops with six loops on a proximal end and six loops on a distal end. Each expansion strut pair loop in an expansion column 49 can include a stair step expansion strut 54 with a stepped-down short segment 58 in a distal end and a short stepped-up segment 60 in a proximal end, and a straight expansion strut 52, in a regularly alternating sequence. Each expansion strut pair loop in an expansion column 48 can include a stair step expansion strut with a stepped-down short segment 56 in a proximal end and a short stepped-up segment 62 in a distal end, and a straight expansion strut 52, in a regularly alternating sequence. The embodiment of stent 10 of Figure 3 includes six stair step expansion struts 54 in an expansion column 48 or 49. Expansion struts 52 and 54 are conjoined by a joining loop 66 in a proximal end or a joining loop 68 in a distal end.

A transitional slope 74 can be between a stepped up proximal segment 60 and a straight segment in a stair step expansion strut 54. Likewise, a transitional slope 76 can be between a stepped up distal segment 62 and a straight segment in a stair step expansion strut 54. A transitional slope 70 can be between a stepped down proximal segment 56 and a straight segment in a stair step expansion strut 54. Likewise, a transitional slope 72 can be between a stepped down distal segment 58 and a straight segment in a stair step expansion strut 54.

In an expansion column 48 or 49, a straight segment of expansion strut 52 can have a longitudinal axis 80. Similarly, a stair step expansion strut 54 can have a longitudinal axis 82. Expansion columns 48 and 49 can be horizontally aligned along the axis of the stent, with proximal peaks 66 of expansion strut pair loops of one expansion column 48 aligned with proximal peaks 66 of expansion strut pair loops of adjacent expansion column 49. Short stepped down segments 56 and 58 of adjacent expansion columns 48 and 49 are aligned on the ipsilateral, or same sides. Short stepped up segments 74 and 76 of adjacent expansion columns 48 and 49 are aligned on the ipsilateral, or same sides. Similarly, long straight segments of expansion struts 54 in an adjacent expansion column 48 can also be aligned on the ipsilateral sides.

Figure 5 shows a magnified view of a middle section of one embodiment of a stent 10. A connecting strut has a longitudinal axis 112 or 110. A stair-step connecting strut in a connecting strut column 92 or 94 can have a short segment, for example 100, on an end directly extending from an expansion strut pair of an expansion column and a longer segment, for example 102, on the other end directly extending from an expansion strut pair of an adjacent expansion column. Between these two end segments a connecting strut can include a straight center segment, for example 104, having a slant-angle orientation relative to the two end segments. A central intermediate segment 104 can be placed in close proximity to an expansion strut pair loop of adjacent expansion columns, which can be conjoined by the connecting strut.

A stair-step connecting strut 90 can have each end 96 and 98 conjoined on the ipsilateral sides of apposed expansion strut pair loops of adjacent expansion columns 48 and 49. A longitudinal axis 110 or 112 of a stair-step connecting strut 90 can have a diagonal orientation relative to the longitudinal axis 26 of the stent. A diagonally oriented axis of a stair-step connecting strut 90 in one connecting strut column 92 has a first direction 110. A diagonally oriented axis of a stair-step connecting strut 90 in an adjacent connecting strut column 94 has a second direction 112. Axes 110 and 112 of connecting struts 90 in adjacent connecting strut columns can run in opposing directions.

In some embodiments of the stent, a connecting strut can have three straight segments 100, 102, and 104; two pivot points 106 and 108 with two radii of curvature 106 and 108; and two ends 96 and 98 that extend directly from expansion struts 54 of adjacent expansion columns. The pivot points 106 and 108 can serve as flexing points for stent flexibility. Longitudinal axes, for example 115 and 117, of connecting strut segments can run in a same direction as a longitudinal axis of a stair-step expansion strut. A central intermediate segment 104 of a connecting strut may have an axis, for example 118, not parallel with, for example, other segments of the connecting strut. A longitudinal axis 118 of a central intermediate segment 104 of a connecting strut can have a diagonal orientation to longitudinal axes of other segments of the connecting strut 115 and 117.

## Claims

1. A stent in a non-expanded state, comprising:
a first expansion column (48) including individual expansion struts (50) forming a plurality of expansion strut pairs (51), wherein each expansion strut pair is conjoined by a joining strut loop at either a proximal end or a distal end, two adjacent expansion strut pairs (51) share a common strut (90),
a second expansion column (49) including individual expansion struts (50) forming a plurality of expansion strut pairs (51), wherein two adjacent expansion strut pairs (51) share a common strut (90),
a first connecting strut column (92) including a plurality of individual first connecting struts (90) that couple the first and second expansion columns (48, 49), wherein each of an individual first connecting strut (90) includes a proximal section (100) with a longitudinal axis that is parallel with a longitudinal axis of an expansion strut in the first expansion column (48), and a distal section with a longitudinal axis that is parallel with a longitudinal axis of an expansion strut of the second expansion column (49); each first connecting strut (90) in the first connecting strut column has three sections; **characterized in that** each first connecting strut (90) of the first connecting strut column (92) is contralaterally conjoined to the first and second expansion columns (48, 49), wherein proximal peaks (66) of expansion strut pair loops of one expansion column (48) are aligned with proximal peaks (66) of an adjacent expansion column (49).

2. The stent of claim 1, wherein each first connecting strut (90) of the first connecting strut column (92) has a stair-step configuration; or
at least a portion of the first connecting strut (90) of the first connecting strut column (92) has asymmetrical geometric configurations; or
each first connecting strut (90) of the first connecting strut column (92) has at least two pivot points (106, 108), preferably each pivot point includes a radius of curvature.

3. The stent of claim 1, wherein one expansion strut (54) of an expansion strut pair (51) of the first expansion column (48) has a stair-step segment at a proximal end and a stair-step segment at a distal end; optionally, the other expansion strut of the expansion strut pair (51) of the first expansion column is a straight segment.

4. The stent of claim 1, wherein one expansion strut (54) of an expansion strut pair (51) of the second expansion column has a stair-step segment at a distal end and a stair-step segment at a proximal end, preferably the other expansion strut (52) of the expansion strut pair (51) of the second expansion column is a straight segment.

5. The stent of claim 1, wherein at least one of a distal end and a proximal end of each first connecting strut (90) of the first connecting strut column is a direct extension of an expansion strut pair (51) of the first or second expansion column.

6. The stent of claim 1, wherein a terminal end of the proximal section of each first connecting strut (90) in the first connecting column is conjoined to an expansion strut (54) in the first expansion column (48), and a terminal end of the distal section of each first connecting strut (90) is conjoined to an expansion strut (54) in the second expansion column (49).

7. The stent of claim 1, wherein at least one of a distal end and a proximal end of a first connecting strut (90) in the first connecting column is a direct extension of an expansion strut (54) in the first expansion column or the second expansion column.

8. The stent of claim 1, wherein the proximal section of each first connecting strut (90) has a surface that is conjoined to at least one surface of an expansion strut (54) in the first expansion column, and the distal section of each first connecting strut (90) has at least one surface that is conjoined to an end of an expansion strut (54) in the second expansion column; or
the proximal section of each first connecting strut (90) has an edge that is a linear extension of an edge of an expansion strut (54) in the first expansion column, and the distal section of each first connecting strut (90) has an edge that is a linear extension of an edge of an expansion strut (54) in the second expansion column, preferably a strain relief notch (119) is formed where the edge of the proximal section of each first connecting strut (90) in the first connecting strut column is conjoined with the edge of he expansion strut (54) of the first expansion column, and a strain relief notch (119) is formed where the edge of the distal section of each first connecting strut (90) in the first connecting strut column is conjoined with edge of the expansion strut (54) of the second expansion column.

9. The stent of claim 1, wherein the distal section of each first connecting strut (90) of the first connecting strut column (92) has a greater length than its proximal section.

10. The stent of claim 1, wherein a proximal section of each first connecting strut (90) of the first connecting strut column is a direct extension of an expansion strut pair (54) of the first expansion column (48), and a distal section of each first connecting strut (90) of the first connecting strut column is a direct extension of an expansion strut pair (54) of the second expansion column (49).

11. The stent of claim 1, wherein the longitudinal axis of the proximal section of each first connecting strut (90) of the first connecting strut column (92) is non-parallel to the longitudinal axis of its distal section.

12. The stent of claim 1, wherein
each first connecting strut (90) of the first connecting strut column includes an intermediate section (104) coupled to the proximal (100) and distal (102) sections of the first connecting strut (90), preferably the intermediate section (104) of each first connecting strut (90) of the first connecting strut column has a greater length than a length of its proximal section (100), or
at least a portion of the proximal and distal sections of each first connecting strut (90) of the first connecting strut column (92) have a curvi-linear geometric configuration; or
the intermediate section of each first connecting strut (90) of the first connecting strut column (92) has a longitudinal axis that is non-parallel to a longitudinal axis of the stent (10); or
the intermediate section of each first connecting strut (90) of the first connecting strut column has a longitudinal axis that is positioned diagonally relative to a longitudinal axis of the stent (10); or
the intermediate section of each first connecting strut (90) of the first connecting strut column (92) has a longitudinal axis that extends in a vertically diagonal direction relative to a longitudinal axis of the stent (10); or
at least a portion of the intermediate section of each first connecting strut of the first connecting strut column is in close proximity to an expansion strut pair of the first expansion column.

13. The stent of claim 12, wherein close proximity is in the range of 0.0254 to 1.27 mm (0.001 to 0.050 of an inch), preferably 0.0254 to 1.016 mm (0.001 to 0.040 of an inch), more preferably 0.0254 to 0.762 mm (0.001 to 0.030 of an inch).

14. The stent of claim 1, wherein each first connecting strut (90) in the first connecting strut column (92) has the same longitudinal axis, preferably all of the first connecting struts (90) in the first connecting strut column (92) have parallel longitudinal axes.

15. The stent of claim 1, wherein a width of the proximal section of each first connecting strut (90) in the first connecting strut column (92) is less than a width of the expansion strut (54) of the first expansion column (48), and a width of the distal section of each first connecting strut (90) of the first connecting strut column (92) is less than a width of the expansion strut (54) of the second expansion column (49).

16. The stent of claim 1, wherein distal ends of expansion strut pairs (51) of the first expansion column (48) that are coupled to proximal ends of expansion strut pairs (51) of the second expansion column are laterally offset 49.

17. The stent of claim 1, wherein distal ends of expansion strut pairs (51) of the first expansion column (48) that are coupled to proximal ends of expansion strut pairs (51) of the second expansion column (49) are vertically offset.

18. The stent of claim 1, wherein the first and second end expansion columns (48, 49) define a proximal and a distal end of the stent, preferably the first and second end expansion columns (48, 49) are mirror images of each other.

19. The stent of claim 1, wherein the first expansion column (48), the second expansion column (49) and the first connecting strut column (92) define a plurality of cells (34), preferably the plurality of cells (34) have asymmetrical geometries, preferably the plurality of cells (34) have symmetrical geometries; or the plurality of cells (34) have evenly spaced geometric shapes; or
the plurality of cells (34) have evenly spaced geometric shapes, more preferably with a quasi-hexagonal geometry in a nominally expanded state.

20. The stent of claim 1, further comprising:
a third expansion column including individual expansion struts forming a plurality of expansion strut pairs (51), wherein two adjacent expansion strut pairs (51) share a common strut;
a second connecting strut column (94) including a plurality of individual second connecting struts (90) that couple the second (49) and third expansion columns, wherein each of an individual second connecting strut (90) of the second connecting strut column (94) includes a proximal section with a longitudinal axis that is parallel with a longitudinal axis of an expansion strut (54) in the second expansion column (49), and a distal section with a longitudinal axis that is parallel with a longitudinal axis of an expansion strut of the third expansion column, preferably each second connecting strut (90) of the second connecting strut column (94) includes an intermediate section (104) coupled to the proximal and distal sections of the second connecting strut (90); or
each second connecting strut (90) in the second connecting strut column (94) has the same longitudinal axis, preferably all of the second connecting struts (90) in the second connecting strut column (94) have parallel longitudinal axes; or
all of the longitudinal axes first connecting struts (90) in the first connecting strut column (92) extend in an opposing direction to all of the longitudinal axes of the second connecting struts (90) in the second connecting column (94).

21. The stent of claim 20, wherein the intermediate section (104) of each second connecting strut (90) of the second connecting strut column has a longitudinal axis that is positioned in a first diagonal direction relative to a longitudinal axis of the stent, preferably, each first connecting strut (90) of the first connecting strut column (92) has an intermediate section (104) with a longitudinal axis positioned in a second diagonal direction relative to a longitudinal axis of the stent, wherein the first and second diagonal directions extend in opposite directions and are non-parallel; or
the intermediate section (104) of each second connecting strut (90) of the second connecting strut column (92) has a longitudinal axis that extends in a vertically diagonal direction relative to a longitudinal axis of the stent; or
at least a portion of the intermediate section (104) of each second connecting strut (90) of the second connecting strut column is in close proximity to an expansion strut pair (51) of the second expansion column.

22. The stent of claim 21, wherein close proximity is in the range of 0.025 to 1.3 mm (0.001 to 0.050 of an inch), preferably 0.025 to 1 mm (0.001 to 0.040 of an inch), more preferably, 0.025 to 0.8 mm (0.001 to 0.030 of an inch).

23. The stent of claim 20, wherein a width of the proximal section of each second connecting strut (90) in the second connecting strut column (94) is less than a width of the expansion strut (54) of the second expansion column (49), and a width of the distal section of each second connecting strut (90) in the second connecting strut column (94) is less than a width of the expansion strut (54) of the third expansion column.

24. The stent of claim 20, wherein distal ends of expansion strut pairs (54) of the second expansion column (54) that are coupled to proximal ends of expansion strut pairs (51) of the third expansion column are laterally offset.

25. The stent of claim 12, wherein at least a portion of the intermediate section (104) of each first connecting strut of the first connecting strut column has a curvi-linear geometric configuration.

## Patentansprüche

1. Ein Stent in einem nicht-expandierten Zustand, der umfasst:
ein erstes säulenförmiges Expansionsglied (48), das einzelne Expansionsstreben (50) enthält, die eine Vielzahl von Expansionsstrebenpaaren (51) bilden, wobei jedes Expansionsstrebenpaar durch eine verbindende schlaufenförmige Strebe entweder am nahen Ende oder am fernen Ende miteinander verbunden ist,
zwei benachbarte Expansionsstrebenpaare (51), die eine gemeinsame Strebe (90) teilen,
ein zweites säulenförmiges Expansionsglied (49), das einzelne Expansionsstreben (50) beinhaltet, die eine Vielzahl von Expansionsstrebenpaaren (51) bilden, wobei zwei benachbarte Expansionsstrebenpaare (51) eine gemeinsame Strebe (90) teilen,
ein erstes verbindendes säulenförmiges Verstrebungsglied (92), das eine Vielzahl von einzelnen ersten Verbindungsstreben (90) enthält, die die ersten und zweiten säulenförmigen Expansionsglieder (48, 49) miteinander koppeln, wobei jede der einzelnen ersten Verbindungsstreben (90) einen nahen Abschnitt (100) mit einer Längsachse, die parallel zu einer Längsachse einer Expansionsstrebe im ersten säulenförmigen Expansionsglied (48) verläuft, und einen fernen Abschnitt mit einer Längsachse, die parallel zu einer Längsachse einer Expansionsstrebe des zweiten säulenförmigen Expansionsglieds (49) verläuft, umfasst; jede erste Verbindungsstrebe (90) im ersten verbindenden säulenförmigen Verstrebungsglied besitzt drei Abschnitte; **dadurch gekennzeichnet, dass** jede erste Verbindungsstrebe (90) des ersten verbindenden säulenförmigen Verstrebungsglieds (92) seitlich gegenüberliegend mit den ersten und zweiten säulenförmigen Expansionsgliedern (48, 49) verbunden ist, wobei nahe Scheitelpunkte (66) der schlaufenförmigen Expansionsstrebenpaare eines säulenförmigen Expansionsglieds (48) an den nahen Scheitelpunkten (66) eines benachbarten säulenförmigen Expansionsglieds (49) ausgerichtet sind.

2. Der Stent nach Anspruch 1, wobei jede erste Verbindungsstrebe (90) des ersten verbindenden säulenförmigen Verstrebungsglieds (92) eine treppenstufenförmige Struktur besitzt; oder
zumindest ein Teil der ersten Verbindungsstrebe (90) des ersten verbindenden säulenförmigen Verstrebungsglieds (92) eine asymmetrische geometrische Struktur besitzt; oder
jede erste Verbindungsstrebe (90) des ersten verbindenden säulenförmigen Verstrebungsglieds (92) zumindest zwei Wendepunkte (106, 108) besitzt und jeder Wendepunkt möglichst einen Krümmungsradius aufweist.

3. Der Stent nach Anspruch 1, wobei eine Expansionsstrebe (54) eines Expansionsstrebenpaares (51) des ersten säulenförmigen Expansionsglieds (48) ein treppenstufenförmiges Segment an einem nahen Ende und ein treppenstufenförmiges Segment an einem fernen Ende besitzt; wahlweise ist die andere Expansionsstrebe des Expansionsstrebenpaares (51) des ersten säulenförmigen Expansionsglieds (48) ein gerades Segment.

4. Der Stent nach Anspruch 1, wobei eine Expansionsstrebe (54) eines Expansionsstrebenpaares (51) des zweiten säulenförmigen Expansionsglieds ein treppenstufenförmiges Segment an einem fernen Ende und ein treppenstufenförmiges Segment an einem nahen Ende besitzt und vorzugsweise die andere Expansionsstrebe (52) des Expansionsstrebenpaares (51) des zweiten säulenförmigen Expansionsglieds ein gerades Segment ist.

5. Der Stent nach Anspruch 1, wobei zumindest eins von einem fernen Ende und von einem nahen Ende einer jeden ersten Verbindungsstrebe (90) des ersten verbindenden säulenförmigen Verstrebungsglieds ein unmittelbares Ansatzstück eines Expansionsstrebenpaares (51) des ersten oder zweiten Expansionsglieds darstellt.

6. Der Stent nach Anspruch 1, wobei ein abschließendes Ende des nahen Abschnitts einer jeden ersten Verbindungsstrebe (90) im ersten verbindenden säulenförmigen Verstrebungsglied mit einer Expansionsstrebe (54) im ersten säulenförmigen Expansionsglied (48) verbunden ist, und ein abschließendes Ende des fernen Abschnitts einer jeden ersten Verbindungsstrebe (90) mit einer Expansionsstrebe (54) im zweiten säulenförmigen Expansionsglied (49) verbunden ist.

7. Der Stent nach Anspruch 1, wobei zumindest eins von einem fernen Ende und von einem nahen Ende einer ersten Verbindungsstrebe (90) im ersten verbindenden säulenförmigen Verstrebungsglied ein unmittelbares Ansatzstück einer Expansionsstrebe (54) im ersten säulenförmigen Expansionsglied oder im zweiten säulenförmigen Expansionsglied darstellt.

8. Der Stent nach Anspruch 1, wobei der nahe Abschnitt einer jeden ersten Verbindungsstrebe (90) eine Oberfläche besitzt, die mit zumindest einer Oberfläche einer Expansionsstrebe (54) im ersten säulenförmigen Expansionsglied verbunden ist, und der ferne Abschnitt einer jeden ersten Verbindungsstrebe (90) zumindest eine Oberfläche besitzt, die mit einem Ende einer Expansionsstrebe (54) im zweiten säulenförmigen Expansionsglied verbunden ist; oder
der nahe Abschnitt einer jeden ersten Verbindungsstrebe (90) einen Rand besitzt, der ein lineares Ansatzstück eines Randes einer Expansionsstrebe (54) im ersten säulenförmigen Expansionsglied darstellt, und der ferne Abschnitt einer jeden ersten Verbindungsstrebe (90) einen Rand besitzt, der ein lineares Ansatzstück eines Randes einer Expansionsstrebe (54) im zweiten säulenförmigen Expansionsglied darstellt. Vorzugsweise ist eine Zugentlastungskerbe (119) dort ausgebildet, wo der Rand des nahen Abschnitts einer jeden ersten Verbindungsstrebe (90) im ersten verbindenden säulenförmigen Verstrebungsglied mit dem Rand der Expansionsstrebe (54) des ersten säulenförmigen Expansionsglieds verbunden ist, und eine Zugentlastungskerbe (119) ist dort ausgebildet, wo der Rand des fernen Abschnitts einer jeden ersten Verbindungsstrebe (90) im ersten verbindenden säulenförmigen Verstrebungsglied mit dem Rand der Expansionsstrebe (54) des zweiten säulenförmigen Expansionsglieds verbunden ist.

9. Der Stent nach Anspruch 1, wobei der ferne Abschnitt einer jeden ersten Verbindungsstrebe (90) des ersten verbindenden säulenförmigen Verstrebungsglieds (92) eine größere Länge besitzt als ihr naher Abschnitt.

10. Der Stent nach Anspruch 1, wobei ein naher Abschnitt einer jeden ersten Verbindungsstrebe (90) des ersten verbindenden säulenförmigen Verstrebungsglieds ein unmittelbares Ansatzstück eines Expansionsstrebenpaares (54) des ersten säulenförmigen Expansionsglieds (48) darstellt, und ein ferner Abschnitt einer jeden ersten Verbindungsstrebe (90) des ersten verbindenden säulenförmigen Verstrebungsglieds ein unmittelbares Ansatzstück eines Expansionsstrebenpaares (54) des zweiten säulenförmigen Expansionsglieds (49) darstellt.

11. Der Stent nach Anspruch 1, wobei die Längsachse des nahen Abschnitts einer jeden ersten Verbindungsstrebe (90) des ersten verbindenden säulenförmigen Verstrebungsglieds (92) nicht-parallel zur Längsachse ihres fernen Abschnitts verläuft.

12. Der Stent nach Anspruch 1, wobei
jede erste Verbindungsstrebe (90) des ersten verbindenden säulenförmigen Verstrebungsglieds einen dazwischenliegenden Abschnitt (104) enthält, der mit den nahen (100) und fernen (102) Abschnitten der ersten Verbindungsstrebe (90) gekoppelt ist, der dazwischenliegende Abschnitt (104) einer jeden ersten Verbindungsstrebe (90) des ersten verbindenden säulenförmigen Verstrebungsglieds vorzugsweise eine Länge besitzt, die größer ist als die Länge ihres nahen Abschnitts (100), oder
zumindest ein Teil der nahen und fernen Abschnitte einer jeden ersten Verbindungsstrebe (90) des ersten verbindenden säulenförmigen Verstrebungsglieds (92) eine gekrümmte geometrische Struktur besitzt; oder
der dazwischenliegende Abschnitt einer jeden ersten Verbindungsstrebe (90) des ersten verbindenden säulenförmigen Verstrebungsglieds (92) eine Längsachse besitzt, die nicht-parallel zu einer Längsachse des Stents (10) verläuft; oder
der dazwischenliegende Abschnitt einer jeden ersten Verbindungsstrebe (90) des ersten verbindenden säulenförmigen Verstrebungsglieds eine Längsachse besitzt, die sich in einer relativ zu einer Längsachse des Stents (10) diagonalen Richtung erstreckt; oder
der dazwischenliegende Abschnitt einer jeden ersten Verbindungsstrebe (90) des ersten verbindenden säulenförmigen Verstrebungsglieds (92) eine Längsachse besitzt, die sich in einer relativ zu einer Längsachse des Stents (10) vertikal diagonalen Richtung erstreckt; oder
zumindest ein Teil eines dazwischenliegenden Abschnitts einer jeden ersten Verbindungsstrebe des ersten verbindenden säulenförmigen Verstrebungsglieds sich in enger Nachbarschaft zu einem Expansionsstrebenpaar des ersten säulenförmigen Expansionsglieds befindet.

13. Der Stent nach Anspruch 12, wobei die enge Nachbarschaft im Bereich von 0.0254 bis zu 1.27 mm (0.001 bis zu 0.050 inch), vorzugsweise von 0.0254 bis zu 1.016 mm (0.001 bis zu 0.040 inch) und noch bevorzugter von 0.0254 bis zu 0.762 mm (0.001 bis zu 0.030 inch) liegt.

14. Der Stent nach Anspruch 1, wobei jede erste Verbindungsstrebe (90) im ersten verbindenden säulenförmigen Verstrebungsglied (92) die gleiche Längsachse besitzt, und vorzugsweise alle ersten Verbindungsstreben (90) im ersten verbindenden säulenförmigen Verstrebungsglied (92) parallele Längsachsen aufweisen.

15. Der Stent nach Anspruch 1, wobei ein Querschnitt des nahen Abschnitts einer jeden ersten Verbindungsstrebe (90) im ersten verbindenden säulenförmigen Verstrebungsglied (92) kleiner ist als ein Querschnitt der Expansionsstrebe (54) des ersten säulenförmigen Expansionsglieds (48), und ein Querschnitt des fernen Abschnitts einer jeden ersten Verbindungsstrebe (90) im ersten verbindenden säulenförmigen Verstrebungsglied (92) kleiner ist als ein Querschnitt der Expansionsstrebe (54) des zweiten säulenförmigen Expansionsglieds (49).

16. Der Stent nach Anspruch 1, wobei die fernen Enden der Expansionsstrebenpaare (51) des ersten säulenförmigen Expansionsglieds (48), die mit den nahen Enden der Expansionsstrebenpaare (51) des zweiten säulenförmigen Expansionsglieds (49) gekoppelt sind, seitlich versetzt sind.

17. Der Stent nach Anspruch 1, wobei die fernen Enden der Expansionsstrebenpaare (51) des ersten säulenförmigen Expansionsglieds (48), die mit den nahen Enden der Expansionsstrebenpaare (51) des zweiten säulenförmigen Expansionsglieds (49) gekoppelt sind, vertikal versetzt sind.

18. Der Stent nach Anspruch 1, wobei die ersten und zweiten säulenförmigen Expansionsglieder (48, 49) ein nahes und ein fernes Ende des Stents begrenzen, und die ersten und zweiten säulenförmigen Expansionsglieder (48, 49) Spiegelbilder ihrer selbst darstellen.

19. Der Stent nach Anspruch 1, wobei das erste säulenförmige Expansionsglied (48), das zweite säulenförmige Expansionsglied (49) und das erste verbindende säulenförmige Verstrebungsglied (92) eine Vielzahl von Zellen (34) begrenzen, die Vielzahl der Zellen (34) vorzugsweise asymmetrische Geometrien besitzt, die Vielzahl der Zellen (34) vorzugsweise symmetrische Geometrien besitzt; oder
die Vielzahl von Zellen (34) gleichmäßig beabstandete geometrische Formen besitzt; oder
die Vielzahl von Zellen (34) gleichmäßig beabstandete geometrische Formen besitzt, noch bevorzugter mit einer quasihexagonalen Geometrie in einem nominal expandierten Zustand.

20. Der Stent nach Anspruch 1, der weiter umfasst:
ein drittes säulenförmiges Expansionsglied, das einzelne Expansionsstreben beinhaltet, die eine Vielzahl von Expansionsstrebenpaaren (51) bilden, wobei zwei benachbarte Expansionsstrebenpaare (51) eine gemeinsame Strebe teilen;
ein zweites verbindendes säulenförmiges Verstrebungsglied (94), das eine Vielzahl von einzelnen zweiten Verbindungsstreben (90) enthält, die die zweiten (49) und dritten säulenförmigen Expansionsglieder miteinander koppeln, wobei jede der einzelnen zweiten Verbindungsstreben (90) des zweiten verbindenden säulenförmigen Verstrebungsglieds (94) einen nahen Abschnitt mit einer Längsachse enthält, die parallel zu einer Längsachse einer Expansionsstrebe (54) im zweiten säulenförmigen Expansionsglied (49) verläuft, und einen fernen Abschnitt mit einer Längsachse enthält, die parallel zu einer Längsachse einer Expansionsstrebe des dritten säulenförmigen Expansionsglieds verläuft, und vorzugsweise jede zweite Verbindungsstrebe (90) des zweiten verbindenden säulenförmigen Verstrebungsglieds (94) einen dazwischenliegenden Abschnitt (104) beinhaltet, der mit den nahen und fernen Abschnitten der zweiten Verbindungsstrebe (90) gekoppelt ist; oder
jede zweite Verbindungsstrebe (90) im zweiten verbindenden säulenförmigen Verstrebungsglied (94) die gleiche Längsachse besitzt, und vorzugsweise alle zweiten Verbindungsstreben (90) im zweiten verbindenden säulenförmigen Verstrebungsglied (94) parallele Längsachsen besitzen; oder
alle Längsachsen der ersten Verbindungsstreben (90) im ersten verbindenden säulenförmigen Verstrebungsglied (92) sich in die gegenüberliegende Richtung aller Längsachsen der zweiten Verbindungsstreben (90) im zweiten verbindenden säulenförmigen Verstrebungsglied (94) erstrecken.

21. Der Stent nach Anspruch 20, wobei der dazwischenliegende Abschnitt (104) einer jeden zweiten Verbindungsstrebe (90) des zweiten verbindenden säulenförmigen Verstrebungsglieds eine Längsachse besitzt, die relativ zu einer Längsachse des Stents in einer ersten diagonalen Richtung positioniert ist,
und vorzugsweise jede erste Verbindungsstrebe (90) des ersten verbindenden säulenförmigen Verstrebungsglieds (92) einen dazwischenliegenden Abschnitt (104) besitzt, mit einer Längsachse, die relativ zu einer Längsachse des Stents in einer zweiten diagonalen Richtung positioniert ist, wobei sich die ersten und zweiten diagonalen Richtungen entgegengesetzt erstrecken und nicht parallel verlaufen; oder
der dazwischenliegende Abschnitt (104) einer jeden zweiten Verbindungsstrebe (90) des zweiten verbindenden säulenförmigen Verstrebungsglieds (92) eine Längsachse besitzt, die sich relativ zu einer Längsachse des Stents in einer vertikal diagonalen Richtung erstreckt; oder
zumindest ein Teil des dazwischenliegenden Abschnitts (104) einer jeden zweiten Verbindungsstrebe (90) des zweiten verbindenden säulenförmigen Verstrebungsglieds sich in enger Nachbarschaft zu einem Expansionsstrebenpaar (51) des zweiten säulenförmigen Expansionsglieds befindet.

22. Der Stent nach Anspruch 21, wobei die enge Nachbarschaft im Bereich von 0.025 bis zu 1.3 mm (0.001 bis zu 0.050 inch), vorzugsweise von 0.025 bis zu 1 mm (0.001 bis zu 0.040 inch) und noch vorzugsweiser von 0.025 bis zu 0.8 mm (0.001 bis zu 0.030 inch) liegt.

23. Der Stent nach Anspruch 20, wobei ein Querschnitt des nahen Abschnitts einer jeden zweiten Verbindungsstrebe (90) im zweiten verbindenden säulenförmigen Verstrebungsglied (94) kleiner ist als ein Querschnitt der Expansionsstrebe (54) des zweiten säulenförmigen Expansionsglieds (49), und ein Querschnitt des fernen Abschnitts einer jeden zweiten Verbindungsstrebe (90) im zweiten verbindenden säulenförmigen Verstrebungsglied (94) kleiner ist als ein Querschnitt der Expansionsstrebe (54) des dritten säulenförmigen Expansionsglieds.

24. Der Stent nach Anspruch 20, wobei ferne Enden von Expansionsstrebenpaaren (54) des zweiten säulenförmigen Expansionsglieds (54), die mit nahen Enden von Expansionsstrebenpaaren (51) des dritten säulenförmigen Expansionsglieds gekoppelt sind, seitlich versetzt sind.

25. Der Stent nach Anspruch 12, wobei zumindest ein Teil des dazwischenliegenden Abschnitts (104) einer jeden ersten Verbindungsstrebe des ersten verbindenden säulenförmigen Verstrebungsglieds eine gekrümmte geometrische Struktur aufweist.

## Revendications

1. Stent dans un état non expansé, comprenant :
une première colonne d'expansion (48) comprenant des entretoises d'expansion individuelles (50) formant une pluralité de paires d'entretoises d'expansion (51), dans laquelle chaque paire d'entretoises d'expansion est jointe par une boucle d'entretoise de raccordement à une extrémité proximale ou à une extrémité distale, deux paires d'entretoises d'expansion adjacentes (51) partageant une entretoise commune (90),
une deuxième colonne d'expansion (49) comprenant des entretoises d'expansion individuelles (50) formant une pluralité de paires d'entretoises d'expansion (51), dans laquelle deux paires d'entretoises d'expansion adjacentes (51) partagent une entretoise commune (90),
une première colonne d'entretoises de connexion (92) comprenant une pluralité de premières entretoises de connexion individuelles (90) qui couplent la première et la seconde colonnes d'expansion (48, 49), dans laquelle chacune des premières entretoises de connexion individuelles (90) comprend une section proximale (100) ayant un axe longitudinal parallèle à un axe longitudinal d'une entretoise d'expansion dans la première colonne d'expansion (48) et une section distale ayant un axe longitudinal parallèle à un axe longitudinal d'une entretoise d'expansion de la deuxième colonne d'expansion (49) ; chaque première entretoise de connexion (90) dans la première colonne d'entretoises de connexion comporte trois sections ; **caractérisé en ce que** chaque première entretoise de connexion (90) de la première colonne d'entretoises de connexion (92) est jointe de manière contralatérale aux première et seconde colonnes d'expansion (48, 49), dans lequel les crêtes proximales (66) des boucles de paires d'entretoises d'expansion d'une colonne d'expansion (48) sont alignées avec les crêtes proximales (66) d'une colonne d'expansion adjacente (49).

2. Stent selon la revendication 1, dans lequel chaque première entretoise de connexion (90) de la première colonne d'entretoises de connexion (92) a une configuration en marche d'escalier ; ou
au moins une partie de la première entretoise de connexion (90) de la première colonne d'entretoises de connexion (92) a des configurations géométriques asymétriques ; ou
chaque première entretoise de connexion (90) de la première colonne d'entretoises de connexion (92) a au moins deux points pivotants (106, 108), chaque point pivotant comprenant de préférence un rayon de courbure.

3. Stent selon la revendication 1, dans lequel une entretoise d'expansion (54) d'une paire d'entretoises d'expansion (51) de la première colonne d'expansion (48) a un segment en marche d'escalier au niveau d'une extrémité proximale et un segment en marche d'escalier au niveau d'une extrémité distale ; de manière facultative, l'autre entretoise d'expansion de la paire d'entretoises d'expansion (51) de la première colonne d'expansion est un segment droit.

4. Stent selon la revendication 1, dans lequel une entretoise d'expansion (54) d'une paire d'entretoises d'expansion (51) de la deuxième colonne d'expansion a un segment en marche d'escalier au niveau d'une extrémité distale et un segment en marche d'escalier au niveau d'une extrémité proximale, et dans lequel de préférence l'autre entretoise d'expansion (52) de la paire d'entretoises d'expansion (51) de la deuxième colonne d'expansion est un segment droit.

5. Stent selon la revendication 1, dans lequel au moins une extrémité parmi une extrémité distale et une extrémité proximale de chaque première entretoise de connexion (90) de la première colonne d'entretoises de connexion est une extension directe d'une paire d'entretoises d'expansion (51) de la première ou de la deuxième colonne d'expansion.

6. Stent selon la revendication 1, dans lequel une extrémité terminale de la section proximale de chaque première entretoise de connexion (90) dans la première colonne de connexion est jointe à une entretoise d'expansion (54) dans la première colonne d'expansion (48) et une extrémité terminale de la section distale de chaque première entretoise de connexion (90) est jointe à une entretoise d'expansion (54) dans la deuxième colonne d'expansion (49).

7. Stent selon la revendication 1, dans lequel au moins une extrémité parmi une extrémité distale et une extrémité proximale d'une première entretoise de connexion (90) dans la première colonne de connexion est une extension directe d'une entretoise d'expansion (54) dans la première colonne d'expansion ou la deuxième colonne d'expansion.

8. Stent selon la revendication 1, dans lequel la section proximale de chaque première entretoise de connexion (90) a une surface qui est jointe à au moins une surface d'une entretoise d'expansion (54) dans la première colonne d'expansion et la section distale de chaque première entretoise de connexion (90) a au moins une surface qui est jointe à une extrémité d'une entretoise d'expansion (54) dans la deuxième colonne d'expansion ; ou
la section proximale de chaque première entretoise de connexion (90) a un bord qui est une extension linéaire d'un bord d'une entretoise d'expansion (54) dans la première colonne d'expansion et la section distale de chaque première entretoise de connexion (90) a un bord qui est une extension linéaire d'un bord d'une entretoise d'expansion (54) dans la deuxième colonne d'expansion, de préférence une entaille de décharge de traction (119) est formée à l'endroit où le bord de la section proximale de chaque première entretoise de connexion (90) dans la première colonne d'entretoises de connexion est joint au bord de l'entretoise d'expansion (54) de la première colonne d'expansion et une entaille de décharge de traction (119) est formée à l'endroit où le bord de la section distale de chaque première entretoise de connexion (90) dans la première colonne d'entretoises de connexion est joint au bord de l'entretoise d'expansion (54) de la deuxième colonne d'expansion.

9. Stent selon la revendication 1, dans lequel la section distale de chaque première entretoise de connexion (90) de la première colonne d'entretoises de connexion (92) a une longueur supérieure à sa section proximale.

10. Stent selon la revendication 1, dans lequel une section proximale de chaque première entretoise de connexion (90) de la première colonne d'entretoises de connexion est une extension directe d'une paire d'entretoises d'expansion (54) de la première colonne d'expansion (48) et une section distale de chaque première entretoise de connexion (90) de la première colonne d'entretoises de connexion est une extension directe d'une paire d'entretoises d'expansion (54) de la deuxième colonne d'expansion (49).

11. Stent selon la revendication 1, dans lequel l'axe longitudinal de la section proximale de chaque première entretoise de connexion (90) de la première colonne d'entretoises de connexion (92) n'est pas parallèle à l'axe longitudinal de sa section distale.

12. Stent selon la revendication 1, dans lequel :
chaque première entretoise de connexion (90) de la première colonne d'entretoises de connexion comprend une section intermédiaire (104) couplée aux sections proximale (100) et distale (102) de la première entretoise de connexion (90), de préférence la section intermédiaire (104) de chaque première entretoise de connexion (90) de la première colonne d'entretoises de connexion a une longueur supérieure à une longueur de sa section proximale (100) ; ou
au moins une partie des sections proximale et distale de chaque première entretoise de connexion (90) de la première colonne d'entretoises de connexion (92) a une configuration géométrique curviligne ; ou
la section intermédiaire de chaque entretoise de connexion (90) de la première colonne d'entretoises de connexion (92) a un axe longitudinal qui n'est pas parallèle à un axe longitudinal du stent (10) ; ou
la section intermédiaire de chaque première entretoise de connexion (90) de la première colonne d'entretoises de connexion a un axe longitudinal qui s'étend dans une direction diagonale par rapport à un axe longitudinal du stent (10) ; ou
la section intermédiaire de chaque première entretoise de connexion (90) de la première colonne d'entretoises de connexion (92) a un axe longitudinal qui s'étend dans une direction verticalement diagonale par rapport à un axe longitudinal du stent (10) ; ou
au moins une partie de la section intermédiaire de chaque première entretoise de connexion de la première colonne d'entretoises de connexion est à proximité d'une paire d'entretoises d'expansion de la première colonne d'expansion.

13. Stent selon la revendication 12, dans lequel la proximité est comprise dans la plage de 0,0254 à 1,27 mm (0,001 à 0,050 pouce), de préférence de 0,0254 à 1,016 mm (0,001 à 0,040 pouce), plus préférablement de 0,0254 à 0,762 mm (0,001 à 0,030 pouce).

14. Stent selon la revendication 1, dans lequel chaque première entretoise de connexion (90) dans la première colonne d'entretoises de connexion (92) a le même axe longitudinal, de préférence toutes les premières entretoises de connexion (90) dans la première colonne d'entretoises de connexion (92) ont des axes longitudinaux parallèles.

15. Stent selon la revendication 1, dans lequel une largeur de la section proximale de chaque première entretoise de connexion (90) dans la première colonne d'entretoises de connexion (92) est inférieure à une largeur de l'entretoise d'expansion (54) de la première colonne d'expansion (48) et une largeur de la section distale de chaque première entretoise de connexion (90) de la première colonne d'entretoises de connexion (92) est inférieure à une largeur de l'entretoise d'expansion (54) de la deuxième colonne d'expansion (49).

16. Stent selon la revendication 1, dans lequel les extrémités distales des paires d'entretoises d'expansion (51) de la première colonne d'expansion (48) qui sont couplées aux extrémités proximales des paires d'entretoises d'expansion (51) de la deuxième colonne d'expansion sont latéralement décalées 49.

17. Stent selon la revendication 1, dans lequel les extrémités distales des paires d'entretoises d'expansion (51) de la première colonne d'expansion (48) qui sont couplées aux extrémités proximales des paires d'entretoises d'expansion (51) de la deuxième colonne d'expansion (49) sont verticalement décalées.

18. Stent selon la revendication 1, dans lequel la première et la seconde colonnes d'expansion d'extrémité (48, 49) définissent une extrémité proximale et distale du stent, la première et la seconde colonnes d'expansion d'extrémité (48, 49) étant de préférence des images inverses l'une de l'autre.

19. Stent selon la revendication 1, dans lequel la première colonne d'expansion (48), la deuxième colonne d'expansion (49) et la première colonne d'entretoises de connexion (92) définissent une pluralité de cellules (34), la pluralité de cellules (34) ayant de préférence des géométries asymétriques, la pluralité de cellules (34) ayant de préférence des géométries symétriques ; ou
la pluralité de cellules (34) ayant des formes géométriques espacées de manière homogène ; ou
la pluralité de cellules (34) ayant des formes géométriques espacées de manière homogène, plus préférablement avec une géométrie quasi-hexagonale dans un état nominalement expansé.

20. Stent selon la revendication 1, comprenant en outre :
une troisième colonne d'expansion comprenant des entretoises d'expansion individuelles formant une pluralité de paires d'entretoises d'expansion (51), dans lequel deux paires d'entretoises d'expansion adjacentes (51) partagent une entretoise commune ;
une seconde colonne d'entretoises de connexion (94) comprenant une pluralité de secondes entretoises de connexion individuelles (90) qui couplent les deuxième (49) et troisième colonnes d'expansion, dans lequel chacune des secondes entretoises de connexion individuelles (90) de la seconde colonne d'entretoises de connexion (94) comprend une section proximale ayant un axe longitudinal parallèle à l'axe longitudinal d'une entretoise d'expansion (54) dans la deuxième colonne d'expansion (49) et une section distale ayant un axe longitudinal parallèle à un axe longitudinal d'une entretoise d'expansion de la troisième colonne d'expansion, de préférence chaque seconde entretoise de connexion (90) de la seconde colonne d'entretoises de connexion (94) comprenant une section intermédiaire (104) couplée aux sections proximale et distale de la seconde entretoise de connexion (90) ; ou
chaque seconde entretoise de connexion (90) dans la seconde colonne d'entretoises de connexion (94) a le même axe longitudinal, de préférence toutes les secondes entretoises de connexion (90) dans la seconde colonne d'entretoise de connexion (94) ayant des axes longitudinaux parallèles ; ou
tous les axes longitudinaux des premières entretoises de connexion (90) dans la première colonne d'entretoises de connexion (92) s'étendent dans une direction opposée à tous les axes longitudinaux des secondes entretoises de connexion (90) dans la seconde colonne de connexion (94).

21. Stent selon la revendication 20, dans lequel la section intermédiaire (104) de chaque seconde entretoise de connexion (90) de la seconde colonne d'entretoises de connexion a un axe longitudinal qui est placé dans une première direction diagonale par rapport à un axe longitudinal du stent, de préférence, chaque première entretoise de connexion (90) de la première colonne d'entretoises de connexion (92) a une section intermédiaire (104) ayant un axe longitudinal placé dans une seconde direction diagonale par rapport à un axe longitudinal du stent, dans lequel la première et la seconde directions diagonales s'étendent dans des directions opposées et ne sont pas parallèles ; ou
la section intermédiaire (104) de chaque seconde entretoise de connexion (90) de la seconde colonne d'entretoises de connexion (92) a un axe longitudinal qui s'étend dans une direction verticalement diagonale par rapport à un axe longitudinal du stent ; ou
au moins une partie de la section intermédiaire (104) de chaque seconde entretoise de connexion (90) de la seconde colonne d'entretoises de connexion est à proximité d'une paire d'entretoises d'expansion (51) de la deuxième colonne d'expansion.

22. Stent selon la revendication 21, dans lequel la proximité est dans la plage de 0,025 à 1,3 mm (0,001 à 0,050 pouce), de préférence de 0,025 à 1 mm (0,001 à 0,040 pouce), plus préférablement de 0,025 à 0,8 mm (0,001 à 0,030 pouce).

23. Stent selon la revendication 20, dans lequel une largeur de la section proximale de chaque seconde entretoise de connexion (90) dans la seconde colonne d'entretoises de connexion (94) est inférieure à une largeur de l'entretoise d'expansion (54) de la deuxième colonne d'expansion (49) et une largeur de la section distale de chaque seconde entretoise de connexion (90) dans la seconde colonne d'entretoises de connexion (94) est inférieure à une largeur de l'entretoise d'expansion (54) de la troisième colonne d'expansion.

24. Stent selon la revendication 20, dans lequel les extrémités distales des paires d'entretoises d'expansion (54) de la deuxième colonne d'expansion (54) qui sont couplées aux extrémités proximales des paires d'entretoises d'expansion (51) de la troisième colonne d'expansion sont latéralement décalées.

25. Stent selon la revendication 12, dans lequel au moins une partie de la section intermédiaire (104) de chaque première entretoise de connexion de la première colonne d'entretoises de connexion a une configuration géométrique curviligne.
